Europäisches Patentamt

European Patent Office

Office européen des brevets

(19)

(11) Publication number: **0 028 804**
**B1**

(12) **EUROPEAN PATENT SPECIFICATION**

(45) Date of publication of patent specification: **02.05.84**

(51) Int. Cl.³: **A 61 K 39/225**

(21) Application number: **80106801.6**

(22) Date of filing: **05.11.80**

(54) Vaccine and method for its preparation.

(30) Priority: **13.11.79 US 93070**

(43) Date of publication of application:
**20.05.81 Bulletin 81/20**

(45) Publication of the grant of the patent:
**02.05.84 Bulletin 84/18**

(84) Designated Contracting States:
**AT BE CH DE FR GB IT LI LU NL SE**

(56) References cited:
**GB - A - 1 508 050**
**US - A - 3 479 430**

(73) Proprietor: **Iowa State University Research Foundation, Inc.,**
**315 Beardshear, Iowa State University Ames Iowa 50010 (US)**

(72) Inventor: **Gough, Patricia Marie**
**R.R. 2**
**Ames Iowa 50010 (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath Maximilianstrasse 58**
**D-8000 München 22 (DE)**

Courier Press, Leamington Spa, England.

Vaccine and method for its preparation

Transmissible gastroenteritis (TGE) of swine is caused by a coronavirus (TGEV). The disease occurs wherever swine are raised, and it is highly fatal for baby pigs during the first few weeks after farrowing. However, if the sow has had a recent TGE infection, protective antibodies are transferred to the offspring in the colostrum and milk which protect the baby pigs against TGE. Bohl et al, *Infect. & Immun.*, 6, 289—230 (1972). TGE vaccines have been developed which are capable of producing high serum virus neutralizing titers to TGEV. Such vaccines are prepared from attenuated, non-virulent TGEV, strains of which may be prepared by continuous cell culture of virulent TGEV. See, for example, United States patents 3,479,430 and 3,585,108. However, such vaccines are not very effective in producing lactogenic immunity in the sow, whether the attenuated TGEV is given live or inactivated. Tamoglia, *J.A.V.M.A.*, 160, 554—558 (1972).

Studies have shown that IgA antibodies are produced in milk by TGE infection or by administration of live virulent TGEV, while the administration of attenuated vaccines produces mainly IgG antibodies. Bohl et al, *Infect. & Immun.*, 11, 23—32 (1975). Bohl et al suggest that effective passive immunization of baby pigs may depend on continual neutralization of virus within their intestinal tracts by the IgA antibodies produced in milk after exposure of the sow to virulent TGE. However, vaccines containing virulent TGEV would be hazardous to use, since the virus is readily transferred among swine in a herd.

Coronaviruses have been reported as containing from four to sixteen different proteins, which are in the form of polypeptides, some of which are glycosylated. Wege et al, *J. Gen. Virol.* 42, 37—47 (1979). For example, Wege et al analyzed the proteins of the murine coronavirus and found that it contained six proteins, two of which were glycoproteins, and that the protein components ranged in molecular weight from about 170,000 to 22,700. A preliminary report on the purification and fractionation of the proteins of TGE virus was presented by Gough and Ellis to the 1976 Conference of Research Workers in Animal Disease at Chicago, Illinois. An Abstract of the Gough and Ellis paper was published. Abstracts of Papers Presented at Conference of Research Workers in Animal Disease, 57th Annual Meeting Nov. 29—30, 1967, p. 11.

This invention is based in part on the discovery that an important antigenic factor is at least partially lost in the recovery of TGE virus for use in vaccines. This antigenic factor appears to be a protein associated with the surface of the corona of the virus from which it is easily separated by mechanical attrition, such as would occur when the TGE virus is subjected to sonication during a recovery procedure. For the purpose of the present invention, this antigenic factor is designated as "TGE-SPA" or as "SPA", since it is believed to be a specific protein antigen for protection of swine against TGE. The quantity of the SPA factor associated with TGEV appears to be reduced by attenuation, that is, virulent strains of TGE are believed to contain relatively more of the SPA than do attenuated strains. Possibly, the failure of attenuated strain vaccines to produce the same level of lactogenic immunity as obtained by natural TGE infection or by experimental injection of virulent TGEV in breeding sows is related to this difference.

The present invention is further based on the findings that the TGE-SPA for use in preparing vaccines in accordance with the present invention may be readily separated from TGEV or other sub-viral protein components of TGE, and that the SPA may be identified by physical criteria which characterize specific protein molecules, such as buoyant density and electrophoretic mobility. This SPA factor and the vaccines which can be prepared therefrom will now be described in detail.

The TGE-SPA immunogen for use in the present invention is prepared from TGEV, which has been replicated in the small intestines of baby pigs or in pig cell culture. Since virulent strains of TGEV appear to contain more of the desired specific antigen, it is preferred to use virulent TGEV. However, the SPA can also be obtained from TGEV which has been adapted to cell culture. If the virus is to be propagated in cell culture it may be advantageous to use the adapted, less virulent virus. In general, however, it is preferred to infect the cells with TGEV which is capable of causing TGE in swine. For example, the Illinois isolant of TGEV may be used as the virus for replication either *in vivo* in the pig intestine, or *in vitro* in a culture of swine cells such as testes cells. The Illinois isolant is a standard laboratory virus, and can be obtained from the Veterinary Services Laboratory, U.S.D.A., Ames, Iowa, and other sources. Other suitable virulent isolants can readily be obtained from the intestines of pigs naturally infected with TGE.

The TGEV is isolated from the culture medium. For example, the jejunum of the small intestines of the pig may be subdivided, such as by grinding, and a suspension formed therefrom in a suitable aqueous medium. Similarly, the cultured cells may be suspended in a suitable medium for further processing. The suspension is subjected to centrifugation to remove the intestinal or cellular debris. The supernatant contains the TGEV. The virus is precipitated from the supernatant by a suitable precipitating agent such as polyethylene glycol or ammonium sulfate. On completion of the precipitation, the precipitate is collected by centrifugation, for example, at 9500 g for 30 minutes. The separated precipitate is then redispersed in a suitable aqueous medium for further purification. For example, this can be accomplished by rate-zonal contrifugation through a discontinuous sucrose density gradient. The buoyant density of the TGEV precipitated with polyethylene glycol 6000 is 1.122 gm/ml. The virus will therefore sediment through 10, 25, and 40% discontinuous sucrose gradients to the 25—40% sucrose interface.

The virus-containing band is separated. At this point, the desired SPA is still associated with the TGEV, apparently being a constituent of the surface of the TGE virion. To liberate the SPA, mechanical energy may be applied to the virus. One preferred procedure is to subject the TGEV suspension to sonication. As the SPA is broken off it goes into solution. The desired viral protein is therefore characterized in one respect by its water solubility.

The sonicated suspension of the TGEV may contain two viral proteins in solution both of which have buoyant densities of 1.02 to 1.03 grams per milliliter. These proteins can therefore be recovered separately from the residual TGEV by isopycnic centrifugation using a continuous sucrose density gradient. For example, the isopycnic centrifugation may be carried out using a gradient of 10 to 60% sucrose for 18—20 hours at 260,000 g. The second band of material, which may be referred to as "Band B", corresponding to about 6 to 8% sucrose, will contain the SPA together with the other protein component. The separated Band B may be used directly to form a vaccine in accordance with the present invention, or it may be further purified to increase the concentration of the SPA relative to the other protein.

For example, on a laboratory basis, the Band B protein solution may be further purified by gel filtration using a colum of Sepharose® 4B or Sephadex® G200. Applying a tris-saline buffer as the eluant, the desired immunogen (TGE-SPA) will be the second fraction to be eluted, the first fraction containing the other protein of similar buoyant density. Where this concentration is above that desired for the vaccine, the eluate may be diluted to the concentration for preparing the vaccine. Alternatively, where the concentration of the eluate is below that desired for the vaccine, the SPA may be concentrated by procedures such as electrophoresis, ultrafiltration, endosmosis or precipitation and resuspension such as with polyethylene glycol or other precipitating agents.

For commercial production, techniques other than isopycnic centrifugation and gel filtration may be employed for isolating the SPA. For example, in a simplified commercial procedure the precipitated TGE virus would be resuspended in an aqueous medium subjected to mechanical or chemical treatment to liberate the SPA (viz. sonication, enzymic hydrolysis, disruption with detergents, etc.), and then processed by ultrafiltration for molecular size separation. The SPA is the smallest solubilized protein molecule passing through the pores of the filter membrane and is recovered in the filtrate. The residual viral particles and most of the other protein of similar buoyant density can thereby be separated from the SPA.

The SPA protein which is used as the immunogen in vaccines of the present invention has been further characterized by its electrophoretic mobility, which is referred to as the Rf value. The Rf value is determined under charge-neutralized conditions, such as in the presence of sodium dodecyl sulfate, and is relative to human serum albumin by polyacrylamide gel electrophoresis. Under these conditions, the Rf value of the SPA protein is $1.84 \pm .01$. The other protein of Band B, which has the same buoyant density, has a similarly determined Rf value of $1.40 \pm .01$. Molecular weights calculated from these Rf values give an approximate molecular weight for the SPA protein of 25,000, and for the other Band B protein of 32,000.

As described, the SPA immunogen prepared in accordance with the present invention is free of TGE virus. It may be in admixture with the other viral protein of the same buoyant density, as obtained in the form of Band B. However, further purification is desirable so that the final vaccine immunogen is composed substantially entirely of the SPA protein. Preferably, 90% or more of the protein of the immunogen may be the SPA protein which is water-soluble, has a buoyant density of 1.02 to 1.03 gm/ml, and a charge-neutralized electrophoretic mobility (Rf value) of $1.84 \pm .01$ relative to human serum albumin.

The SPA immunogen can be prepared in vaccine dose form by well known procedures. For parenteral administration, such as intramuscular injection, the SPA is combined with a suitable adjuvant. Aluminium hydroxide has been found to be a desirable adjuvant. Aluminium hydroxide adjuvants can be prepared as described in U.S. patent No. 3,149,036, or obtained from a commercial source, such as Merck Adjuvant 65, or Merck & Co., Inc. Rahway, N.J., U.S.A. Other standard adjuvants can also be used such as Freund's Incomplete Adjuvant, which can be obtained from Difco Laboratories, Detroit, Michigan, U.S.A. The relative proportions of SPA immunogen and adjuvant can be varied, providing both are present in effective amounts. For example, the aluminium hydroxide adjuvant may be present in an amount of 0.5% of the vaccine mixture ($Al_2O_3$ basis). On a per dose basis, the concentration of the SPA immunogen may range from 0.25 to 4.0 mg, but is preferably within the range from about 0.5 to 3.0 mg per dose. For example, good results are obtained with intramuscular administration of 1.0 to 2.0 mg of SPA per dose to pregnant sows for protection of the baby pig offspring. A convenient dose size is about one milliliter. Therefore, a representative dose for intramuscular injection would comprise 1 ml containing 1.0 mg of SPA in admixture with 0.5% aluminium hydroxide adjuvant. Similar dose forms may also be prepared for parenteral administration to baby pigs, but the amount of SPA given per dose may be smaller such as 0.25 to 1.0 mg per dose. The SPA immunogen may also be prepared in the form of an enteric-coated oral vaccine, for example, as described in U.S. patent 3,823,228.

The mechanism of action of the SPA vaccines is not fully understood. For passive immunization of baby pigs, it appears that the pregnant sow is stimulated to produce the required immunizing antibodies in the colostrum and milk. However, because of the small size of the SPA molecule, it is possible that some of the antigen is transferred through the placenta to the baby pigs, thereby stimulating direct production of immunity in the baby pigs prior to or immediately following birth. Not only is the vaccine highly effective for such passive immunization but it has the advantage of being entirely safe to use. There is no possibility of TGE infection being caused by the vaccine, as where live virulent TGE virus are used. Further, as compared with TGE vaccines prepared from attenuated non-virulent strains of TGEV, the SPA vaccines are much more effective for passive immunization of baby pigs. Another important advantage of the SPA vaccines is that they can be used as part of a program for TGE eradication. The antibodies produced by SPA vaccination can be distinguished from the antibodies produced by natural TGE infection, and therefore infected swine can be distinguished from immunized vaccinated swine.

The invention and the results which can be obtained thereby are further illustrated by the following examples.

### Example I

The TGE-SPA immunogen for use in preparing vaccines in accordance with the present invention may be prepared as follows:

1. The TGEV is produced from the virulent Illinois isolant of TGEV, which is replicated in small intestines of baby SPF pigs (infected at 48 hrs of age, virus generally harvested 48 hrs post-infection). A 20% suspension of the ground jejunum of the injected pigs is made in minimum essential medium (Eagle's) to which 5% lactalbumin hydrolysate, 2% fetal porcine serum and antibiotics have been added. Alternatively, the Illinois isolant is replicated in roller cultures of swine testes cells. The cells are 6-day-old (post-passage) monolayers when infected and harvest of virus is done at 3 days post-inoculation. Higher titers of virus are obtained with older cells (6 to 8 days) than with younger (3 to 5 days) cells. This is especially important with strains of TGE virus (such as Illinois) which are not well-adapted to *in vitro* conditions. Virus is collected after two freeze-thaw cycles of cells-medium.

2. The crude virus is centrifuged at approximately 5000 g for 20 to 30 minutes to remove cellular or intestinal debris.

3. The virus is precipitated from the supernatant by adding 7% polyethylene glycol 6000 and 2.3% sodium chloride. The mixture is held at 40°C for 1.5 to 2.5 hours for precipitation to be completed.

4. The precipitate is collected by centrifugation at 9500 g for 30 min. The precipitate is dispersed in Eagle's minimum essential medium, pH 7.0, to a volume 1/20th of the original.

5. The dispersion is subjected to rate-zonal centrifugation through a discontinuous (10, 25, 40% sucrose) sucrose density gradient (in tris-EDTA buffered saline at pH 7.4) at 100,000 g for 1.5 hrs. Buoyant density of the virus is 1.122 gm/ml and it sediments to the 25—40% sucrose interface.

6. The virus-containing band is separated and sonicated, using a Biosonic IV Sonic Oscillator for 10 sec. at low power, thereby breaking off viral protein from the TGEV. (The separated protein dissolves in the water phase). The suspension may then be dialyzed against tris-EDTA buffered saline (pH 7.4) to remove sucrose.

7. The suspension containing the solubilized viral protein is subjected to isopycnic centrifugation through a continuous (10 to 60% sucrose) sucrose density gradient for 18—20 hours at 260,000 g. The TGE virus has a buoyant density of 1.18 gm/ml (approximately 42% sucrose). The desired protein band ("Band B") has a buoyant density of 1.02 to 1.03 gm/ml (6 to 8% sucrose). The Band B protein is recovered as an aqueous solution and used to prepare the vaccine.

8. The Band B solution is analyzed by nitrogen determination to obtain total protein content, so that the protein concentration can be adjusted to the level desired for vaccine preparation, such as approximately 1 mg/ml of total protein. This corresponds to approximately 0.5 mg/ml of SPA, which comprises about 50% of the Band B protein. The sucrose may be left in the solution or removed by dialysis. If the solution is to be frozen for storage the sucrose will act as a cryoprotectant.

9. As required, the protein concentration of the Band B solution can be increased by dialysis of the solution against polyethylene glycol, or decreased by addition of tris-saline buffer to give a concentration of approximately 1 mg/ml of total protein.

10. The Band B protein solution obtained in step 7 may be separated and further purified by gel filtration, using Sephadex® G200. The gel filtration may be carried out with upward flow at a flow rate of 6 ml/hour using a tris-saline buffer. Two components are obtained as separate eluate fractions. The SPA is the second fraction to be eluted, and is the smaller of the two protein molecules contained in Band B. By polyacrylamide gel electrophoresis in the presence of sodium dodecyl sulfate, according to a standard proceeding described in "Bishob and Roy J. Virology 10 p. 234—243 1972, the SPA protein has an Rf value of 1.84 $\pm$ .01 (relative to human serum albumin). The other protein of Band B (which also has a buoyant density of 1.02 to 1.03 gm/ml) has a similarly determined Rf value of 1.40 $\pm$ .01. The calculated molecular weight of the SPA immunogen is approximately 25,000, and the other Band B viral protein has a calculated molecular weight of approximately 32,000. The isolated SPA, free of the other Band B protein, may be used to prepare vaccines.

### Example II

To prepare an intramuscularly injectable vaccine from the Band B protein of Example I or the isolated SPA protein, the viral protein is suspended in an aqueous solution of an aluminium hydroxide adjuvant. The vaccine should contain 1.0 milligrams of Band B protein per milliliter, or 0.5 mg/ml of SPA. The aluminium hydroxide concentration can be adjusted to approximately 0.5% ($Al_2O_3$ basis). At the indicated concentrations of the Band B and SPA protein, a dose of approximately 2 milliliters should be used, thereby administering approximately 1.0 mg of SPA per dose.

For vaccination of sows, a two dose regimen can be used. The first dose may be given from several months to five to seven weeks prior to farrowing. For example, all of the sows may be vaccinated at one time in the Fall, such as around the first week in November. The second dose of the vaccine should be administered several weeks after the first dose, such as two to four weeks later, and may be given up to the time of farrowing. Alternatively, the SPA immunogen may be given as a single 2 ml dose, such as five to seven weeks prior to farrowing. However, a two-dose regimen is believed preferable for the most effective passive immunization of the baby pigs. All administrations may be given by intramuscular injection.

### Example III

For experimental purposes, Band B protein prepared as described in Example I was combined with Freund's complete adjuvant, using equal parts of the adjuvant and an aqueous solution of the Band B protein containing approximately 0.5 mg of SPA protein per ml. The Band B protein solution was also combined with an aluminium hydroxide adjuvant to prepare a vaccine containing approximately 0.5 mg of SPA protein per ml with an aluminium hydroxide concentration of 0.5% ($Al_2O_3$ basis). Both vaccines were used in approximately 2 ml dose amounts, in vaccine trials as summarized below.

*Vaccine trials.* Data from two vaccination trials with the TGE-SPA immunogen in bred sows are presented. In the first, one sow was immunized with the product administered with Freund's complete adjuvant initially, and with Freund's incomplete adjuvant in two booster inoculations. This animal farrowed three times. The second trial included four gilts immunized with TGE-SPA immunogen in the aluminium hydroxide adjuvant. These animals farrowed only twice.

1. Sow 831—Freund's complete adjuvant.

A duroc first-litter gilt, seronegative for TGE, was immunized with 2 ml (approximately 1 mg) of TGE-SPA immunogen in Freund's complete adjuvant 6 weeks prior to farrowing. Two weeks prior to farrowing she received a booster inoculation of TGE-SPA immunogen in Freund's incomplete adjuvant.

a. Trial 1, 1st farrowing. The sow farrowed 13 piglets; all were very small. On the day following farrowing, the TGE antibody titer of her milk was 534; a serum titer was 178.

One-half of the piglets was orally challenged with 1 ml of Illinois TGE virus (50—100 $PID_{50}$) at 3 days of age; the other half was to acquire the disease naturally by exposure to infected littermates. None of the pigs showed signs of clinical TGE at any time after challenge, but all had serum antibodies when killed at $4\frac{1}{2}$ months of age; no latent virus was detected at that time. Control pigs died of TGE following oral challenge.

b. Trial 2, 2nd farrowing. The sow was not immunized again prior to a second farrowing. At that time she had a serum TGE antibody of 38. Her colostral TGE antibody titer was 125 and that of her milk, 3 days later, was 34. The five piglets all developed clinical signs of TGE upon challenge with 50—100 $PID_{50}$ Illinois TGE virus. Two of the pigs (40%) died of the disease.

c. Trial 3, 3rd farrowing. One month prior to farrowing the sow was given a booster immunization of TGE-SPA immunogen in Freund's incomplete adjuvant. Her serum titer at this time was 33. A granuloma appeared at the injection site. At the time of farrowing the serum TGE antibody titer was 231 and colostral antibody titer was greater than 625 (highest dilution). The antibody titer in milk at 5 days post-farrowing was 700.

None of the seven piglets in the litter developed clinical signs of TGE following oral challenge with 50—100 $PID_{50}$ Illinois isolant of TGE virus. Within 2 days after challenge of the piglets, the granuloma at the injection site of the sow had doubled in size.

Since no pigs had developed clinical TGE within 3 days after challenge with 50—100 $PID_{50}$, one piglet was given $5 \times 10^4$ $PID_{50}$ of Illinois isolant of TGE virus on the fourth day post-challenge. Clinical signs of TGE were not observed in either the challenged pig or in littermates. Diarrhea developed in some of the piglets prior to weaning (at about 2 weeks of age). The diarrhea was not typical of TGE, no other signs of disease were apparent and TGE virus could not be isolated from or identified in diarrhetic feces or from the animals themselves. Bacteria (flagellated short rods) were observed in the feces at copious concentration.

2. Aluminium hydroxide adjuvant. Six crossbred first-litter gilts, seronegative for TGE virus, were used in this experiment. Four were vaccinated at 5 weeks pre-farrowing with 2 mg of TGE-SPA immunogen aluminium hydroxide (0.5% $Al_2O_3$) and two served as controls. A second dose of immunogen was given to the four previously vaccinated gilts 2 weeks prior to farrowing. All of the piglets were challenged orally with 50—100 $PID_{50}$ of the Illinois isolant of TGE virus as a 10% gut slurry at 1 to 3 days post-farrowing.

The piglets on the two control gilts, were showing signs of infection with TGE virus at 32 hours post-challenge. There was 100% morbidity in the two control litters. Three of nine pigs in one litter and one of eight in the other survived the infection, for a mortality rate of 76%.

No clinical signs of TGE were observed in piglets on two of the four vaccinated gilts. These litters consisted of nine piglets challenged at 1 day of age (Gilt 290) and six piglets challenged at 3 days of age (Gilt 294) with 50—100 $PID_{50}$ of Illinois isolant of TGE virus.

Mild clinical signs of TGE (e.g. light diarrhea) did develop in piglets of the remaining two litters on vaccinated gilts after the pigs were challenged with Illinois TGE virus (50—100 $PID_{50}$ at 3 (Gilt 291) and 2 (Gilt 433) days of age. The disease was characterized by light diarrhea in the nine piglets, appearing 56 hours post challenge. However, only one piglet died of TGE. (Three piglets died from causes unrelated to TGE infection). Two pigs died from causes other than TGE.

### Example IV

For a large scale commercial production of vaccines prepared in accordance with the present invention, the procedure of Example I can be followed, using a continuous flow density gradient centrifuge for the separation of step 7 to obtain the Band B protein. Alternatively, other protein separation and purification procedures can be used, such as ultrafiltration, reverse osmosis, and selective precipitation.

In applying an ultrafiltration technique, for example, the resuspended precipitate obtained in step 4 of Example I may be subjected to sonication as described in step 6 (omitting step 5). The resulting sonicated aqueous suspension may then be subjected to ultrafiltration. The Band B protein may be recovered by using hollow fibers or a membrane having a porosity permitting proteins of a size below 50,000 molecular weight to pass therethrough, thereby recovering both proteins of Band B in the filtrate. The ultrafiltration apparatus of Amicon Corporation, Lexington, Mass. may be used. With the hollow fiber type apparatus, the Diaflo® fibers H10 x 50 can be used. For recovering the SPA protein separately from the other Band B protein, the Amicon thin-channel apparatus may be used, selecting a membrane with a 30,000 molecular weight cut-off, such as WM30 or PM30. The SPA protein will be collected in the filtrate, while the other Band B protein will be retained.

### Example V

In another procedure, steps 1 to 4 of Example I are carried out. The resuspended precipitate of the TGE virus is then treated with a non-ionic detergent to extract the protein. For example, Triton® X—100 or Nonidet® P40 can be added to the suspension to a concentration of 0.1% for the extraction. The resulting extract can then be processed by isopycnic centrifugation as described in step 7 of Example I, or by alternative protein recovery procedures described in Example IV. Triton® X—100 is produced by Rohm & Haas Co., Philadelphia, Penn., USA, and is distributed by Sigma Chemical Co., St. Louis, Mo. USA, and Nonidet P40 is produced by BDH Chemicals Ltd., Poole, England. The ability of such non-ionic detergents in dilute solution to solubilize viral protein is well known.

As used herein, "polyethylene glycol 6000" refers to polyethylene glycol having a molecular weight of from 6000 to 7500.

### Claims

1. A vaccine for protecting swine against transmissible gastroenteritis (TGE) infection, comprising a swine-administrable form of vaccine essentially free of TGE virus, containing a specific antigenic protein (SPA) obtained by separation from TGE virus, which SPA is characterized by (i) being soluble in water, (ii) having a buoyant density of 1.02 to 1.03 grams per milliliter, and (iii) having a charge-neutralized electrophoretic mobility (Rf value) of 1.84 ± .01 relative to human serum albumin.

2. The vaccine of claim 1 in which said SPA is obtained from a virulent strain of TGE virus.

3. The vaccine of claim 1 or 2 in which said vaccine is completely free of TGE virus.

4. The vaccine of any one of claims 1 to 3 in which said vaccine is free of TGE viral protein of different buoyant density than said 1.02 to 1.03 gm/ml.

5. The vaccine of any one of claims 1 to 4 in which the TGE-derived protein in said vaccine is composed substantially entirely of said SPA together with another viral protein of the same buoyant density.

6. The vaccine of any one of claims 1 to 4 in which the TGE-derived protein in said vaccine is composed substantially entirely of said SPA.

7. The vaccine of any one of claims 1 to 6, in a swine-administrable dose form.

8. The vaccine of any one of claims 1 to 7 comprising an injectable dose form of an adjuvant in admixture with the specific antigenic protein (SPA) as defined in claim 1, each dose of said vaccine containing from 0.25 to 4.0 mg of said SPA.

9. The parenteral vaccine of claim 8 in which each dose of said vaccine contains from 0.5 to 3.0 mg of said SPA.

10. A process for preparing a vaccine useful for protecting swine against transmissible gastroenteritis (TGE) infection, characterised in that the specific antigenic protein (SPA), as defined in claim 1, is brought into swine-administrable form.

**Revendications**

1. Vaccin pour protéger le porc contre l'infection par la gastroentérite transmissible (TGE) comprenant une forme de vaccin administrable au porc, pratiquement exempt de virus de la TGE, contenant une protéine antigène spécifique (SPA) obtenue par séparation du virus de la TGE, laquelle SPA est caractérisée en ce que (1) elle est soluble dans l'eau, (2) elle a une densité flottante de 1,02 à 1,03 g/ml, et (3) elle a une mobilité électrophorétique à charge-neutralisée (Rf) de $1,84 \pm 0,01$ par rapport à la sérum albumine humaine.

2. Vaccin suivant la revendication 1, caractérisé en ce que cette SPA est obtenue à partir d'une souche virulente de virus de la TGE.

3. Vaccin suivant l'une quelconque des revendications 1 ou 2, dans lequel ce vaccin est complètement exempt de virus de la TGE.

4. Vaccin suivant l'une quelconque des revendications 1 à 3, dans lequel ce vaccin est exempt de protéine virale de la TGE d'une densité flottante différente de ces 1,02 à 1,03 g/ml.

5. Vaccin suivant l'une quelconque des revendications 1 à 4, dans lequel la protéine dérivée de la TGE dans ce vaccin est composée presque intégralement de cette SPA avec une autre protéine virale de la même densité flottante.

6. Vaccin suivant l'une quelconque des revendications 1 à 4, dans lequel la protéine dérivée de la TGE dans ce vaccin est composée presque entièrement de cette SPA.

7. Vaccin suivant l'une quelconque des revendications 1 à 6, sous une forme de dose administrable au porc.

8. Vaccin suivant l'une quelconque des revendications 1 à 7, comprenant une forme de dose injectable d'un adjuvant en mélange avec la protéine antigène spécifique (SPA) telle que définie dans la revendication 1, chaque dose de ce vaccin contenant de 0,25 à 4,0 mg de cette SPA.

9. Vaccin parentéral suivant la revendication 8, dans lequel chaque dose de ce vaccin contient de 0,5 à 3,0 mg de cette SPA.

10. Procédé de préparation d'une vaccin utile pour protéger le porc contre l'infection par la gastroentérite transmissible (TGE), caractérisé en ce que la protéine antigène spécifique (SPA) telle que définie dans la revendication 1, est amenée sous une forme administrable au porc.

**Patentansprüche**

1. Impfstoff zum Schützen von Schweinen gegen übertragbare Gastroenteritis (TGE)-Infektion, umfassend eine für Schweine verabreichbare Form eines im wesentlichen vom TGE-Virus freien Impfstoffs, der ein spezifisches, als Antigen wirkendes Protein (SPA), das durch Abtrennung aus TGE-Virus erhalten wird, enthält, wobei das SPA dadurch gekennzeichnet ist, daß es (i) in Wasser löslich ist, (ii) eine Auftriebsdichte von 1,02 bis 1,03 g/ml und (iii) eine ladungsneutralisierte elektrophoretische Beweglichkeit (Rf-Wert) von $1,84 \pm 0,01$, bezüglich menschlichem Serumalbumin, besitzt.

2. Impfstoff nach Anspruch 1, dadurch gekennzeichnet, daß das SPA aus einem virulenten TGE-Virus-Stamm erhalten wird.

3. Impfstoff nach Ansprüche 1 oder 2, dadurch gekennzeichnet, daß der Impfstoff vollständig frei von TGE-Virus ist.

4. Impfstoff nach einem der Ansprüche 1 bis 3, dadurch gekennzeichnet, daß der Impfstoff frei von TGE-Virus-Protein mit einer anderen Auftriebsdichte als 1,02 bis 1,03 g/ml ist.

5. Impfstoff nach einem der Ansprüche 1 bis 4, dadurch gekennzeichnet, daß das von TGEstammende Protein in dem Impfstoff sich im wesentlichen überwiegend aus dem SPA zusammen mit einem anderen Virus-Protein der gleichen Auftriebsdichte zusammensetzt.

6. Impfstoff nach einem der Ansprüche 1 bis 4, dadurch gekennzeichnet, daß das von TGEstammende Protein in dem Impfstoff sich im wesentlichen überwiegend aus dem SPA zusammensetzt.

7. Impfstoff nach einem der Ansprüche 1 bis 6 in einer für Schweine verabreichbaren Dosisform.

8. Impfstoff nach einem der Ansprüche 1 bis 7, umfassend eine injizierbare Dosisform eines Adjuvans in Beimischung mit dem spezifischen, als Antigen wirkenden Protein (SPA), wie in Anspruch 1, definiert, wobei jede Dosis des Impfstoffs 0,25 bis 4,0 mg des SPA enthält.

9. Parenteral verabreichbarer Impfstoff nach Anspruch 8, wobei jede Dosis des Impfstoffs 0,5 bis 3,0 mg des SPA enthält.

10. Verfahren zur Herstellung eines zum Schützen von Schweinen gegen übertragbare Gastroenteritis (TGE)-Infektion geeigneten Impfstoffs, dadurch gekennzeichnet, daß das spezifische, als Antigen wirkende Protein (SPA), wie in Anspruch 1 definiert, in eine für Schweine verabreichbare Form gebracht wird.